# EUROPEAN PATENT APPLICATION

(11) **EP 4 273 448 A1**
(43) Date of publication of application: **08.11.2023**
(21) Application number: 21913666.0
(22) Date of filing: 26.11.2021
(51) Int. Cl.: F22B 35/00, A61B 18/04

(54) **VAPOR ABLATION APPARATUS, AND HEATING CONTROL METHOD, CONTROLLER, APPARATUS, AND MEDIUM THEREOF**

(30) Priority: 31.12.2020 CN 202011637660
(71) Applicant: Hangzhou Broncus Medical Co., Ltd., Hangzhou, Zhejiang 310051 (CN)
(72) Inventor: TANG, Bixiang, Hangzhou, Zhejiang 310051 (CN); XU, Hong, Hangzhou, Zhejiang 310051 (CN); HUANG, Siyuan, Hangzhou, Zhejiang 310051 (CN)
(74) Representative: Petraz, Gilberto Luigi
(86) International application number: PCT/CN2021/133677
(87) International publication number: WO 2022/142925

(57) **Abstract**

Provided are a vapor ablation apparatus, and heating control method, controller, apparatus, and medium thereof; the heating control method includes: monitoring the current water level, current pressure, and current in-generator temperature of a steam generator; controlling a heating apparatus according to the current water level, the current pressure, and the current in-generator temperature. The disclosure is capable of accurately, promptly, and effectively automatically meeting the needs of a steam generator during heating, and the control results have good stability.

## Description

### TECHNICAL FIELD

The disclosure relates to the field of control of medical apparatus, in particular to a steam ablation apparatus and heating control method, controller, apparatus and medium thereof.

### DESCRIPTION OF THE PRIOR ART

Steam ablation is an emerging technology that forms high-temperature steam and then applies high-temperature steam to the target site in the patient's body. It can be used for local tissue inflammation, injury repair, etc. Steam ablation can be applied, for example, to the bronchi, but is not limited thereto.

The steam ablation apparatus can be provided with a steam generator. During the steam ablation and its preparation process, the steam generator needs to be heated. In the related art, the steam generator is heated by manual manipulation, for example, by manually turning on or off the heating device. Therefore, the manual manipulation has a low efficiency, and the heating effect depends on the operator's experience and response during manipulation, which is not stable.

### SUMMARY OF THE DISCLOSURE

### TECHNICAL PROBLEM

The present disclosure provides a steam ablation apparatus and heating control method, controller, apparatus and medium thereof to solve the problem of low efficiency of manual manipulation, and that the heating effect depends on the operator's experience and response during manipulation, which is not stable.

### TECHNICAL SOLUTION

According to a first aspect of the present disclosure, a heating control method for steam ablation apparatus is provided, which is applied to a control device of the steam ablation apparatus. The steam ablation apparatus includes a steam generator and a heating device, and the heating device is capable of being controlled by the control device and heat the steam generator when the heating device is controlled to be turned on;
The heating control method includes:
Monitoring a current water level in the steam generator, a current pressure and a current temperature in the generator; and
Controlling the heating device according to the current water level, the current pressure and the current temperature in the generator.

In the present disclosure, since the factors for the control of the heating device include the current water level, the current temperature in the generator and the current pressure, the steam generator can be heated according to the current actual water level, temperature in the generator and pressure, so that the actual heating requirements can be timely and accurately met. Further, the heating device is automatically controlled by the control device, without relying on manual manipulation and with a high efficiency, and the control is stable, regardless of the condition, knowledge and experience of the operator. Therefore, the present disclosure can automatically meet the heating requirements for the steam generator accurately, timely and efficiently, and the control has a better stability.

Optionally, the step of controlling the heating device according to the current water level, the current pressure and the current temperature in the generator specifically includes:
Controlling the heating device to be turned on or off according to the current water level, and a specified anti-dry-burning water level and / or minimum normal water level; and
Controlling a heating power of the heating device according to the current pressure and the current temperature in the generator after the heating device is turned on.

In the above optional solution, since the heating will affect the pressure and the temperature in the generator, the heating power can be controlled in a closed cycle based on the current pressure and the current temperature in the generator, which allows the heating power to accurately reach the target (e.g. target pressure and target temperature in the generator in a further optional solution).

Optionally, the step of controlling the heating device to be turned on or off according to the current water level, and the specified anti-dry-burning water level and / or minimum normal water level specifically includes:
Turning on the heating device if the current water level is higher than or equal to the specified minimum normal water level; and
Turning off the heating device if the current water level is lower than the minimum normal water level and higher than or equal to the anti-dry-burning water level.

In the above optional solution, by turning off the heating device in time when the current water level is lower than the minimum normal water level, continuous heating causing the water level to rapidly drop to the anti-dry-burning water level and thus resulting in potential safety hazards can be avoided.

Optionally, the heating device includes a plurality of heaters;
The step of controlling the heating power of the heating device according to the current pressure and the current temperature in the generator specifically includes:
Controlling the turned-on heating device to heat with a target power when the current pressure or the current temperature in the generator falls within a set first range; the target power matching a heating power when all heaters of the heating device are turned on; and
Adjusting the heating power of the heating device according to the current temperature in the generator, the current pressure and set target parameters when the current pressure or the current temperature in the generator falls within a second range, wherein values in the second range are greater than the first range, the target parameters comprise a target temperature and a target pressure, and the target temperature or the target pressure falls within the second range.

In the above optional solution, when the current pressure or the current temperature in the generator is at a small value (such as the first range), all heaters can be controlled to be turned on to reach a higher heating power (such as the maximum heating power), facilitating reaching a greater value (such as the second range) close to the target parameter as soon as possible, and when reaching this range, fine control can be achieved based on the target temperature and target pressure. It can be seen that the above process meets both the efficiency and accuracy requirements for heating.

Optionally, before the step of controlling the heating device to be turned on or off according to the current water level, and the specified anti-dry-burning water level and / or minimum normal water level, the method further includes:

Determining that the steam ablation apparatus enters a target operating state being any one of a preheating state, a standby state, and an ablation preparation state.

In the above optional solution, the heating can be started in time after entering the target operating state, for example, the preheating state, standby state and ablation preparation state that need to be heated. The target operating state is switched automatically, and the processing efficiency is improved.

Optionally, the heating control method further includes monitoring a current steam temperature of the steam generator;
Before the step of determining that the steam ablation apparatus enters the target operating state, the method further includes:
Determining that the steam ablation apparatus has met requirements for the target operating state according to at least one of the current water level, the current steam temperature and the current pressure, and the current operating state of the steam ablation apparatus.

The above optional solution provides a basis for the automatic switch of the state of the steam ablation apparatus, and thus the processing step can be automatically switched, so that the apparatus can enter the corresponding state in a timely and accurate manner for corresponding processing, ensuring the efficiency of the whole process.

Optionally, if the target operating state is the preheating state:
The step of determining that the steam ablation apparatus has met requirements for the target operating state according to at least one of the current water level, the current steam temperature and the current pressure, and the current operating state of the steam ablation apparatus specifically includes:
Detecting that the current water level of the steam generator meets requirements for preheating when the steam ablation apparatus is in a filling state.

The step of detecting that the current water level of the steam generator meets requirements for preheating specifically includes:
Detecting that the current water level is higher than the specified minimum normal water level.

In the above optional solution, it is determined whether the water level required for preheating is met, thus providing a basis for the switch of the operating state, and a relatively sufficient amount of water can be ensured when the subsequent heating is started.

Optionally, if the target operating state is the standby state:
The step of determining that the steam ablation apparatus has met requirements for the target operating state according to at least one of the current water level, the current steam temperature and the current pressure, and the current operating state of the steam ablation apparatus specifically includes:
Detecting that the current water level and the current steam temperature of the steam generator meet requirements for standby when the steam ablation apparatus is in the preheating state.

Optionally, the step of detecting that the current water level and the current steam temperature of the steam generator meet requirements for standby includes:
Detecting that the current water level is higher than or equal to a specified maximum normal water level, and the current steam temperature is higher than a set disinfection temperature threshold.

In the above optional solution, it is determined whether the water level and steam requirements for standby are met, thus providing a basis for the switch of the operating state. Also, a sufficient amount of water (that is, higher than the maximum normal water level) and disinfection temperature during subsequent standby are ensured. Based on the water amount, the steam with a required pressure and the supply of the steam in the subsequent process can be ensured. Based on the disinfection temperature, disinfection during standby can be achieved.

Optionally, if the target operating state is the ablation preparation state:
The step of determining that the steam ablation apparatus has met requirements for the target operating state according to at least one of the current water level, the current steam temperature and the current pressure, and the current operating state of the steam ablation apparatus specifically includes:
Detecting that the current water level of the steam generator meets requirements for steam ablation and the steam ablation apparatus has been disinfected when the steam ablation apparatus is in the standby state.

Optionally, the step of detecting that the current water level of the steam generator meets requirements for steam ablation includes:
Detecting that the current water level is higher than or equal to a specified maximum normal water level.

In the above alternatives, it is determined whether the water level required for steam ablation and disinfection are met, thus providing a basis for the switch of the operating state. Also, a sufficient amount of water (that is, higher than the maximum normal water level) and safety during subsequent ablation are ensured.

Optionally, the step of controlling the heating device to be turned on according to the current water level and the specified anti-dry-burning water level specifically includes:
Turning on the heating device when the current water level is higher than or equal to the anti-dry-burning water level and the current temperature in the generator is higher than a set heat-discharging temperature threshold.

Optionally, the heating device includes a plurality of heaters;
The step of controlling the heating power of the heating device according to the current pressure and the current temperature in the generator specifically includes:
Controlling the turned-on heating device to heat with a target power matching a heating power when all heaters of the heating device are turned on;
Controlling the heating device to keep heating with the target power when the current pressure is lower than a set heat-discharging pressure threshold and the current pressure or the current temperature in the generator falls within a set first range;
Adjusting the heating power of the heating device according to the current temperature in the generator, the current pressure and set target parameters when the current pressure is lower than the set heat-discharging pressure threshold and the current pressure or the current temperature in the generator falls within a set second range; values in the second range are greater than the first range, the target parameters are the target temperature and target pressure in the generator, and the target temperature in the generator or the target pressure falls within the second range.

Optionally, before the step of controlling the heating device to be turned on according to the current water level and the specified anti-dry-burning water level, the method further includes:
Determining that the steam ablation apparatus enters a shutdown operation state.

In the above optional solution, heating can be performed in the shutdown state, so as to meet the requirement of heat-discharging (discharging the water in the steam generator by heating) in the shutdown state, so that the water in the steam generator can be discharged quickly in the shutdown state.

Optionally, the steam ablation apparatus has a plurality of operating states, and the plurality of operating states comprise filling state, preheating state, standby state, ablation preparation state and shutdown operation state;
The filling state refers to the state of filling the steam generator with water but without preheating;
The preheating state refers to the state of preheating the internal environment of the steam generator;
The standby state refers to the state where the steam ablation apparatus is disinfected and the steam generator meets the requirements for steam ablation;
The ablation preparation state refers to the state where the steam generator can always meet the requirements for steam ablation.

In the above technical solution, the specific definition and implementation of the states allow the steam generator to gradually form and maintain the steam required for ablation based on the principle of steam formation, so as to meet the steam ablation requirements, and provide basis for achieving the whole process automatically and gradually.

According to a second aspect of the present disclosure, a heating controller for steam ablation apparatus is provided, which is applied to the control device of the steam ablation apparatus, the steam ablation apparatus includes a steam generator and a heating device, and the heating device is configured to be controlled by the control device and heat the steam generator when the heating device is controlled to be turned on;
The heating controller includes:
A monitoring module, configured to monitor a current water level in the steam generator, a current pressure and a current temperature in the generator; and
A heating control module, configured to control the heating device according to the current water level, the current pressure and the current temperature in the generator.

According to a third aspect of the present disclosure, a steam ablation apparatus is provided, including a steam generator, a heating device, and a control device, the heating device is configured to be controlled by the control device and heat the steam generator when the heating device is controlled to be turned on, and the control device is configured to execute the heating control method and the related optional solutions according to the first aspect.

According to a fourth aspect of the present disclosure, an electronic device is provided, including a processor and a memory,
The memory is used to store codes;
The processor is configured to execute the codes in the memory to implement the heating control method and the related optional solutions according to the first aspect.

According to a fifth aspect of the present disclosure, a storage medium is provided, in which a computer program is stored, and when the program is executed by a processor, the heating control method and the related optional solutions according to the first aspect is implemented.

### BRIEF DESCRIPTION OF DRAWINGS

In order to more clearly illustrate the technical solutions of the embodiments of the present disclosure or the prior art, the drawings that need to be referred in the description of the embodiments or the prior art will be briefly introduced in the following. Obviously, the drawings in the following only show some embodiments of the present disclosure. For those skilled in the art, other drawings can also be obtained according to these drawings without any creative effort.
FIG. 1 is a first schematic diagram of the arrangement of a steam ablation apparatus according to an embodiment of the present disclosure;
FIG. 2 is a second schematic diagram of the arrangement of a steam ablation apparatus according to an embodiment of the present disclosure;
FIG. 3 is a third schematic diagram of the arrangement of a steam ablation apparatus according to an embodiment of the present disclosure;
FIG. 4 is a schematic flowchart of a heating control method for a steam ablation apparatus according to an embodiment of the present disclosure;
FIG. 5 is a schematic flowchart of step S22 according to an embodiment of the present disclosure;
FIG. 6 is a schematic flowchart of step S221 and step S223 according to an embodiment of the present disclosure;
FIG. 7 is a schematic flowchart of step S221, step S222 and step S223 according to an embodiment of the present disclosure;
FIG. 8 is a schematic flowchart of step S221 and step S224 according to an embodiment of the present disclosure;
FIG. 9 is a schematic flowchart of step S221, step S224 and step S222 according to an embodiment of the present disclosure;
FIG. 10 is a first schematic diagram of the program module of the heating controller of the steam ablation apparatus according to an embodiment of the present disclosure;
FIG. 11 is a second schematic diagram of the program module of the heating controller of the steam ablation apparatus according to an embodiment of the present disclosure; and
FIG. 12 is a schematic diagram of the arrangement of an electronic device according to an embodiment of the present disclosure.

### DDESCRIPTION OF EMBODIMENTS

The technical solutions according to the embodiments of the disclosure will be clearly and completely described below in conjunction with the drawings according to the embodiments of the disclosure. Apparently, the described embodiments are some of the embodiments of the present disclosure, not all of them. Based on the embodiments of the present disclosure, all other embodiments obtained by those skilled in the art without creative efforts fall within the protection scope of the present disclosure.

The terms "first", "second", "third", "fourth" and the like (if any) used in the description, the claims and the drawings are intended to distinguish similar objects and don't necessarily define a specific order or sequence. It can be understood that the terms so used here are interchangeable in some appropriate cases such that the embodiments of the disclosure described herein can be implemented in sequences other than those illustrated or described herein. Furthermore, the terms "including" and "having", as well as any variations thereof, are intended to define a non-exclusive inclusion. For example, a process, method, system, product or apparatus including a sequence of steps or elements is not necessarily limited to the listed sequence of steps or elements, but may include other sequence of steps or elements not listed here or inherent to the process, method, product or apparatus.

The technical solutions according to the embodiments of the disclosure will be described in detail below. The following specific embodiments may be combined with each other, and the same or similar concepts or processes may not be repeated in some embodiments.

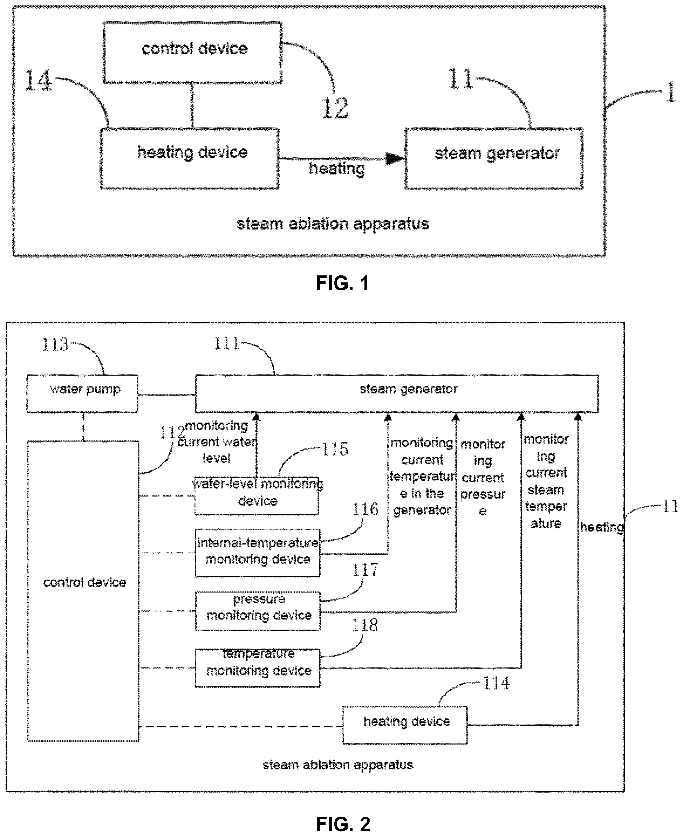

Referring to FIGS. 1 to 3, a steam ablation apparatus 11 according to an embodiment of the present disclosure includes a steam generator 111 and a heating device 114. The heating device 114 is configured to be controlled by the control device 112, and heat the steam generator 111 when it is controlled to be turned on.

The heating device 114 can be any device capable of heating the environment in the steam generator 111. Specifically, the heating device 114 can be fixedly installed in the steam generator 111, for example, and / or be connected to the steam generator through a heat-conducting material. Provided that the steam generator can be heated, no matter how the heating device is assembled or what kind of heating device is used, such heating device does not depart from the scope of the embodiments of the present disclosure. The heating device 114 may be provided at the bottom of the steam generator.

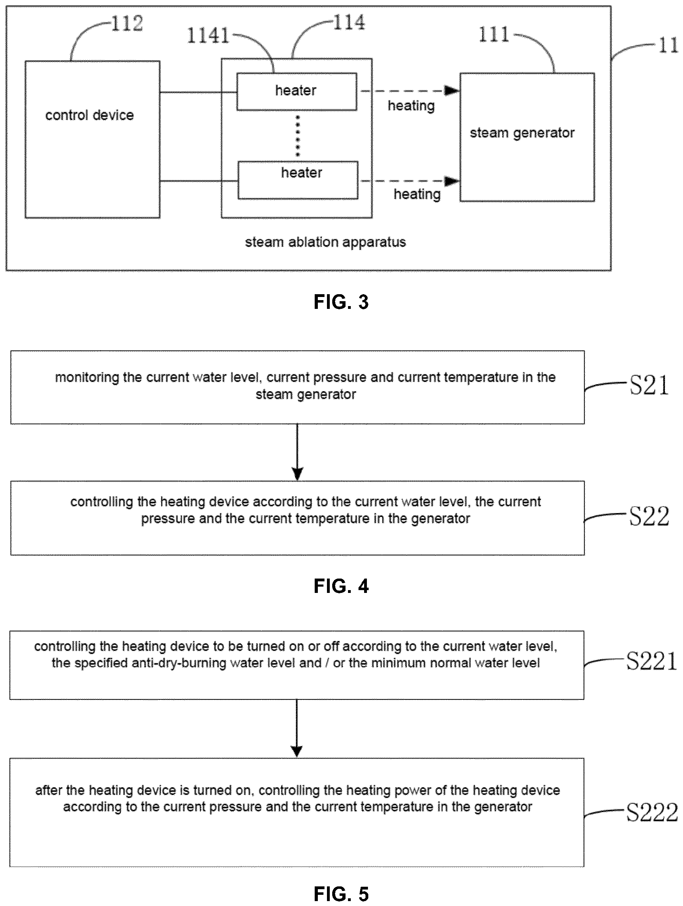

As an example, referring to FIG. 3, the heating device 114 may include at least two heaters 1141 which may be the same or different heaters. For example, the heater 1141 may be a heating rod or a heating ring. At least two heaters 1141 may include at least one heating rod and at least one heating ring. The heating capacity (such as the maximum heating power) of the heating rod can be higher than that of the heating ring, and a corresponding heater can be used for heating as required.

The steam generator 111 can be understood as any device or combination of devices capable of generating steam from the supplied water. For example, it may include a steam generating container for containing water and steam.

In one embodiment, the steam ablation apparatus 2 may further include a water pump 113. The water pump 113 is connected between the water inlet of the steam generator 111 and the water source, and configured to be controlled by the control device 112.

The water source can be any device or combination of devices that can contain water, which can be independent of the steam ablation apparatus and externally connected to the steam ablation apparatus, or can be assembled in the steam ablation apparatus as a part of the steam ablation apparatus.

The water pump 113 can be any device or combination of devices capable of providing a liquid driving force between the water source and the steam generator 111 so that the water from the water source can enter the steam generator 111. The model of the water pump 113 can be varied arbitrarily as required. In some examples, the control device 112 is configured to only switch the water pump 113 on and off. In other examples, in addition to switching the water pump 113 on and off, the control device 112 is further configured to control the driving force of the water pump 113.

Other devices (such as valves, temperature monitoring devices, plasma water monitoring devices, etc.) may be provided between the water pump 113 and the water source, and between the water pump 113 and the steam generator 111.

The water source may be any device or combination of devices that can contain water. In the embodiment shown in FIG. 1, the water source can be independent of the steam ablation apparatus and externally connected to the steam ablation apparatus. In other embodiments, the water source can be assembled in the steam ablation apparatus as a part of the steam ablation apparatus.

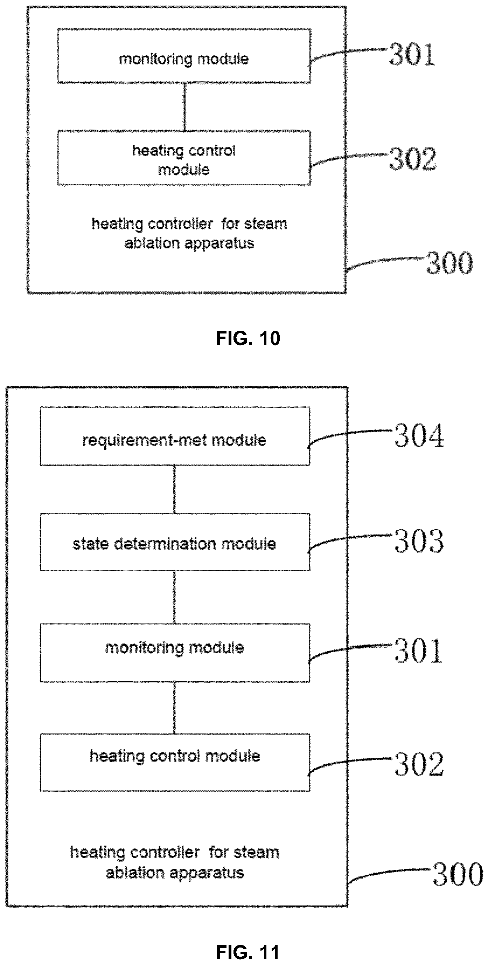

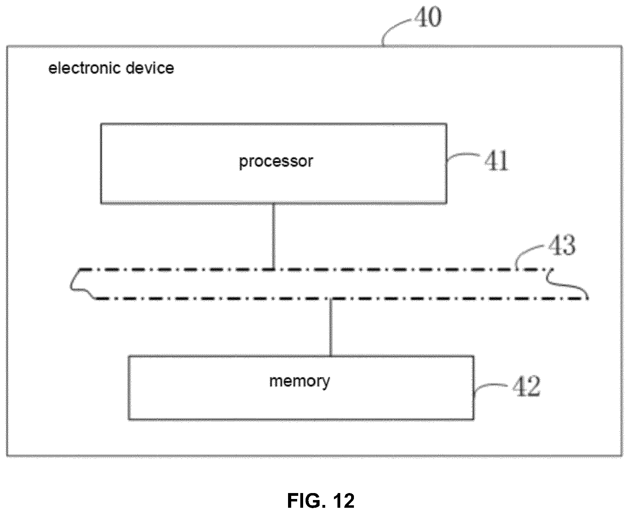

The control device 112 can be any device with data processing capability and communication capability, and the program and / or hardware thereof can be configured arbitrarily based on the water supply control method described in the following. Further, the heating control method as shown in FIGS. 4 to 9 can be implemented only based on the program. That is, the control device 112 is used to execute the heating control method described in the following. Accordingly, a heating controller with various program modules can be provided as shown in FIG. 10. Specifically, the control device 112 may be, for example, the electronic device shown in FIG. 12. In some other examples, at least some of the steps may be implemented through the operation of circuits.

The heating device 114 (and water pump 113, water-level monitoring device 115, internal-temperature monitoring device 116, pressure monitoring device 117, temperature monitoring device 118, etc. shown in FIG. 2) can be in a communication with the control device 112 in a wired or wireless manner. The communication connection may be a direct communication connection or an indirect communication connection, as long as a data interaction with the control device 112 can be realized, which will not depart from the scope of the embodiment of the present disclosure.

Further, in the example as shown in FIGS. 1 to 3, the control device 112 is a part of the steam ablation apparatus 11. In other examples, the control device 112 can be a device independent of the steam ablation apparatus 11. For example, the control device 112 can be a host computer capable of communicating with the steam ablation apparatus 11.

In one embodiment, referring to FIG. 2, the steam ablation apparatus 11 may further include at least one of the followings: a water-level monitoring device 115, an internal-temperature monitoring device 116, a pressure monitoring device 117, an temperature monitoring device 118 and a heating device 114. The monitoring of the current water level, the current temperature in the generator, the current pressure, and the current steam temperature by the control device can be achieved through the water-level monitoring device 115, the internal-temperature monitoring device 116, the pressure monitoring device 117, and the temperature monitoring device 118 respectively.

The water-level monitoring device 115 can be any device capable of monitoring the water level in the steam generator 111. For example, it may have a water-level monitoring container connected with the steam generator 111. The water level in the water-level monitoring container keeps in match with that in the steam generator 111 (at the same level or proportionately). A water-level sensor (such as a float switch) can be provided in the water-level monitoring container. Taking a single float switch as an example, it can monitor whether the water level has reached corresponding one or two water levels, and the corresponding signal is fed back to the control device. The water inlet of the water-level monitoring container can also be connected to the water source through the water pump 113, that is, for example, the water sent by the water pump 113 can enter the steam generator and the water-level monitoring container respectively. However, the embodiments of the present disclosure do not exclude the use of other water-level monitoring devices or water-level sensors, which do not depart from the scope of the embodiments of the present disclosure.

In the following, the monitoring of the current water level may be intended to monitor whether the current water level is higher or lower than the specified water level (such as anti-dry-burning water level, minimum normal water level, maximum normal water level, filling water level, etc.), or to monitor the specific water-level value.

The internal-temperature monitoring device 116 can be any device capable of monitoring the temperature in the steam generator 111. For example, it may include a thermocouple located at the bottom of the steam generator 111, which may measure the water temperature or not. In the following, the monitoring of the temperature in the current generator may be intended to monitor the specific value of the temperature in the generator, or to monitor whether the temperature in the generator reaches the specified temperature value.

The pressure monitoring device 117 can be any device capable of monitoring the steam pressure in the steam generator 111. For example, it may be provided inside the steam generator 111 or outside the steam generator. For example, the pressure monitoring device can be connected to the steam generator through a pipeline, or can be arranged at any outlet or pipeline of the steam generator for steam discharging. In the following, the monitoring of the current pressure may be intended to monitor the specific value of the pressure, or to monitor whether the pressure reaches the specified pressure value.

The temperature monitoring device 118 can be any device capable of monitoring the temperature of the steam in the steam generator 111. For example, it may be provided inside the steam generator 111 or outside the steam generator. For example, the temperature monitoring device can be connected to the steam generator through a pipeline, or can be arranged at any outlet or pipeline of the steam generator for steam discharging. The temperature of the steam measured can be, for example, the temperature of the steam flowing between the steam generator and a steam ablation handle, or can be the temperature of the steam returning from the steam generator to a condensing unit, and the temperature of the steam at the steam ablation handle. In the following, the monitoring of the current steam temperature may be intended to monitor the specific value of the steam temperature, or to monitor whether the steam temperature reaches the specified steam temperature value. The steam temperature may be the same as or different from the temperature in the generator.

Further, the steam ablation apparatus may include the steam ablation handle, the condensing unit, and the like.

The steam ablation handle can be connected to the steam generator through corresponding pipelines and valves, so that the steam from the steam generator can be sent to the steam ablation handle, and then sent to the site to be treated through the steam ablation handle. When the corresponding valves and switches of the steam ablation handle are opened, the steam can be sent out, and when the corresponding valves and switches of the steam ablation handle are closed, the steam can't be sent out from the steam ablation handle.

The condensing unit can be connected to the steam generator through corresponding pipelines and valves so that the steam from the steam generator can be returned to the condensing unit. Further, the steam ablation handle and the condensing unit can be connected to the steam generator through valves with three interfaces (such as three-way valves). The pipelines can be provided with valves (such as solenoid valves) to control the opening and closing of the pipelines. The valves can be controlled by the control device, so that under the control of the control device, the steam generated by the steam generator can optionally enter the outlet of the steam ablation handle or the condensing unit, and it is also possible to control the steam generator to be connected to the outlet of the ablation handle and condensing unit or not (and thus to control the steam to be able to be sent to the outlet of the ablation handle and condensing unit or not).

In some examples, a button switch may be provided on the steam ablation handle, and the button switch may control the discharge of the steam in the steam ablation handle. When the steam needs to be discharged, the user can press the button switch, and the steam will be expelled from the outlet of the steam ablation handle. When the steam is not needed, the user can release the button switch, and the steam will be cut off at the outlet of the ablation handle. In another examples, the button switch or the corresponding valve can be automatically controlled. For example, the user can input corresponding information through a human-computer interaction device, and accordingly the control device can determine the steam ablation time, and automatically control the corresponding switch and/or valve to control the expulsion and cut-off of the steam to meet the steam ablation time.

In one embodiment, the steam ablation apparatus can have a plurality of operating states.

The operating states can be understood as corresponding to different working processes during the steam ablation, and in the respective operating states, the corresponding working processes can be implemented.

The plurality of operating states include at least one of filling state, preheating state, standby state, ablation preparation state, and shutdown operation state. In some examples, all of the filling state, preheating state, standby state, ablation preparation state and shutdown operation state can be provided. In another examples, only part of the operating states can be provided. The steam ablation apparatus can enter the filling state, the preheating state, the standby state and the ablation preparation state sequentially, or enter the filling state, the preheating state, the standby state and the ablation preparation state from other states.

The filling state refers to the state where the steam generator is filled with water but not preheated. Specifically, it refers to the state where the steam generator is filled with water but not preheated after the steam ablation apparatus is powered on and self-tested (or after steam ablation, or after any other state), and the water level in the steam generator is brought to at least the minimum normal water level.

The preheating state refers to the state where the environment in the steam generator is preheated. Specifically, it refers to the state where the environment in the steam generator is preheated after the filling state (or after steam ablation, or after any other state), and the steam in the steam generator exceeds the temperature required for disinfection and the temperature required for steam ablation.

Further, the steam generator may or may not be controlled to communicate with the condensing unit during the filling state and the preheating state.

The standby state refers to the state where the steam generator meets the requirements for steam ablation. Specifically, it refers to the state where the steam generator meets the requirements for steam ablation by performing disinfection on the steam ablation apparatus after the preheating state (or after steam ablation, or after any other state).

Further, during at least part of the disinfection process, the steam generator can be controlled to communicate with the steam ablation handle, so as to send steam (with a temperature higher than the disinfection temperature threshold) to the ablation handle for disinfection. Over a given disinfection time, the disinfection process is completed. After disinfection, the steam generator can be controlled to communicate with the condensing unit, so that steam can be continuously generated and recycled.

The ablation preparation state refers to the state where the steam generator can always meet the steam ablation requirements. Specifically, it refers to the state where the steam generator can always meet the steam ablation requirements after the standby state (or after steam ablation).

Further, when the steam ablation apparatus is in the ablation preparation state, the steam generator can be controlled to communicate with the condensing unit, so that steam can be continuously generated and recycled. In this recycle, the steam required for steam ablation is maintained, and when steam from the steam ablation handle is needed, the steam is discharged from the steam ablation handle, which would not return to the condensing unit.

The steam ablation requirements may include, for example, the disinfection process having been completed and the current water level being higher than the maximum normal water level, and may further include, for example, the interval time from the last steam ablation (steam expulsion) exceeding the time threshold, the current steam temperature exceeding a certain threshold, the current pressure exceeding a certain threshold and so on.

The shutdown operation state refers to the state where the steam ablation apparatus discharges the water and/or steam therein and completes the shutdown of the apparatus. In some examples, the shutdown operation state can be manually or automatically triggered.

In the preheating state, standby state, ablation preparation state and shutdown state, the control device can control the heating device to heat the steam generator. The heating process can refer to the heating control method described in the following.

In the above technical solution, the specific definition and implementation of the states allow the steam generator to gradually form and maintain the steam required for ablation based on the principle of steam formation, so as to meet the steam ablation requirements, and provide basis for achieving the whole process automatically and gradually. The switch of the filling state, the preheating state, the standby state, and the ablation preparation state can be automatically triggered based on the processing in the corresponding state.

The plurality of operating states may further include a power-on self-test state, a shutdown operation state, and the like.

The power-on self-test state refers to the state where the software and hardware of the steam ablation apparatus are self-tested after power-on. In some examples, after the power-on self-test, the steam ablation apparatus can automatically enter the filling state.

The plurality of operating states may further include a monitoring state that can be implemented simultaneously with other states. In the monitoring state, it can be monitored whether various predefined errors occur in the steam ablation apparatus.

The plurality of operating states may further include a configuration state for configuring the software and hardware of the steam ablation apparatus. In the configuration state, relevant personnel can configure the software and hardware of the steam ablation apparatus through the human-computer interaction device or a data transmission medium.

In some examples, the above-mentioned operating states can be represented by state information (such as a specific character or a combination of characters) corresponding to the state. Specifically, the current state of the steam ablation apparatus can be determined (switched or maintained) by setting a specific value or a value at a specific position (which can be considered as a value describing the current operating state of the steam ablation apparatus) as the state information corresponding to the state. For example, the value describing the current operating state of the steam ablation apparatus can be set as the state information of the standby state, indicating that the steam ablation apparatus currently enters or maintains in the standby state.

The specified water level mentioned in the embodiments of the present disclosure may include at least one of the followings: anti-dry-burning water level; minimum normal water level; maximum normal water level; filling water level. The anti-dry-burning water level is lower than the minimum normal water level, the minimum normal water level is lower than the maximum normal water level, and the maximum normal water level is lower than the filling water level.

Among them,
The anti-dry-burning water level reflects the basic requirement for "anti-dry-burning", and provides a basis for judging whether the anti-dry-burning requirement is met during heating the steam generator. Accordingly, the heating control result can meet the anti-dry-burning requirement during heating.

The minimum normal water level and the maximum normal water level reflect the water demand during normal steam ablation and preparation processes, and accordingly, the heating control result can meet the practical requirements.

Referring to FIG. 4, a heating control method of the steam ablation apparatus according to an embodiment of the present disclosure includes:
S21: monitoring the current water level, current pressure and current temperature in the steam generator;
S22: controlling the heating device according to the current water level, the current pressure and the current temperature in the generator.
Steps S21 to S22 are repeated, and can be respectively implemented in different operating states.

In the above technical solution, since the factors for the control of the heating device include the current water level, the current temperature in the generator and the current pressure, the steam generator can be heated according to the current actual water level, temperature in the generator and pressure, so that the actual heating requirements can be timely and accurately met. Further, the heating device is automatically controlled by the control device, without relying on manual manipulation and with a high efficiency, and the control is stable, regardless of the condition, knowledge and experience of the operator. Therefore, the present disclosure can automatically meet the heating requirements for the steam generator accurately, timely and efficiently, and the control has a better stability.

In one embodiment, referring to FIG. 5, step S22 may include:
S221: controlling the heating device to be turned on or off according to the current water level, the specified anti-dry-burning water level and / or the minimum normal water level;
S222: after the heating device is turned on, controlling the heating power of the heating device according to the current pressure and the current temperature in the generator.

In the above optional solution, since the heating will affect the pressure and the temperature in the generator, the heating power can be controlled in a closed cycle based on the current pressure and the current temperature in the generator, which allows the heating power to accurately reach the target (e.g. target pressure and target temperature in the generator in a further optional solution).

The control based on the current pressure and the current temperature in the generator can be implemented based on any logic. Depending on different operating states, the purpose of heating may be different. The heating control implemented may also vary. For example, in target operating states such as preheating state, standby state, and ablation preparation state, one heating control process may be implemented, while in the shutdown operation state, another heating control process may be implemented.

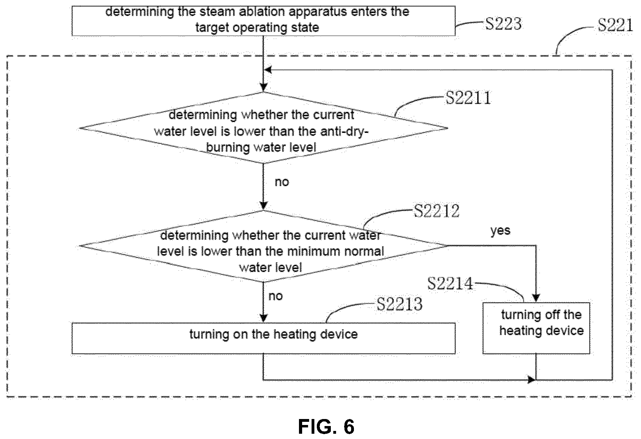

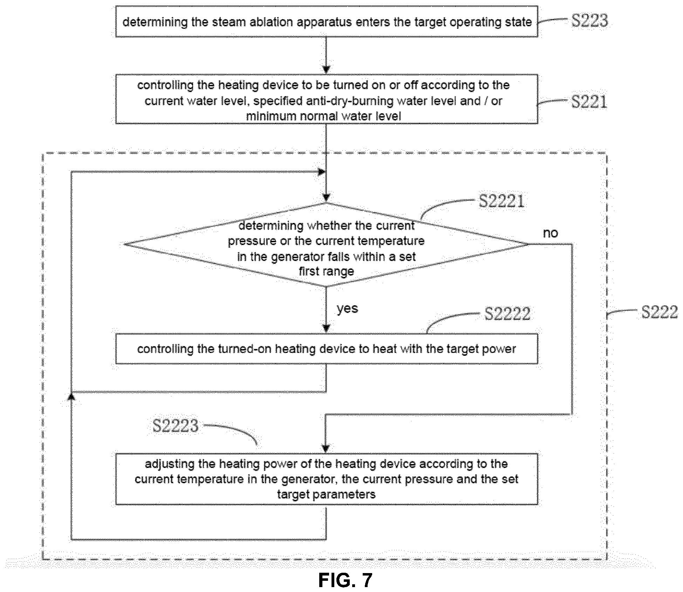

In one embodiment, referring to FIG. 6 and FIG. 7, the specific heating process under the target operating state is shown.

Referring to FIG. 6, step S221 may include:
S2211: determining whether the current water level is lower than the anti-dry-burning water level;
S2212: determining whether the current water level is lower than the minimum normal water level;
If the current water level is higher than or equal to the specified minimum normal water level, step S2213 of turning on the heating device is executed;
If the current water level is lower than the minimum normal water level and higher than or equal to the anti-dry-burning water level, step S2214 of turning off the heating device is executed.

By turning off the heating device in time when the current water level is lower than the minimum normal water level, continuous heating causing the water level to rapidly drop to the anti-dry-burning water level and thus resulting in potential safety hazards can be avoided.

Referring to FIG. 7, step S222 may specifically include:
S2221: determining whether the current pressure or the current temperature in the generator falls within a set first range;
When the current pressure or the current temperature in the generator falls within the set first range, step S2222 of controlling the turned-on heating device to heat with the target power is executed;
When the current pressure or the current temperature in the generator doesn't fall within the first range (for example, falling within a second range), step S2223 of adjusting the heating power of the heating device according to the current temperature in the generator, the current pressure and the set target parameters may be executed. The target parameters include target temperature and target pressure.

The target power is matched with the heating power when all the heaters in the heating device are turned on. When all the heaters are turned on, the heaters can heat with the maximum power or not, and the heating power can vary or not, which all does not depart from the scope of the above technical solution.

Adjusting the heating power according to the target parameters can be achieved by, for example, PID (Proportion Integration Differentiation) control, which can be understood as a proportion-integration-differentiation controller. The feedback loop formed by PID can maintain the stability of the system (that is, the stability of the control result).

The values in the second range are greater than that in the first range, and the target temperature or the target pressure is in the second range. The first range may be, for example, a range less than a certain value which is less than the target temperature, and the second range may be, for example, a range greater than or equal to a certain value which is less than or equal to the target temperature, but they are not limited to the examples above.

In the above optional solution, when the current pressure or the current temperature in the generator is at a small value (such as the first range), all heaters can be controlled to be turned on to reach a higher heating power (such as the maximum heating power), facilitating reaching a greater value (such as the second range) close to the target parameter as soon as possible, and when reaching this range, fine control can be achieved based on the target temperature and target pressure. It can be seen that the above process meets both the efficiency and accuracy requirements for heating.

In one embodiment, since the process shown in FIG. 6 and FIG. 7 are implemented in the target operating state, before step S221, the process may further include:
S223: determining the steam ablation apparatus enters the target operating state.

In the above optional solution, the heating can be started in time after entering the target operating state, for example, the preheating state, standby state and ablation preparation state that need to be heated. The target operating state is switched automatically, and the processing efficiency is improved.

In a further solution, before step S223, the process may further include:
According to at least one of the current water level, the current steam temperature, and the current pressure, and the current operating state of the steam ablation apparatus, determining the steam ablation apparatus has met the requirements for the target operating state.

The above optional solution provides a basis for the automatic switch of the state of the steam ablation apparatus, and thus the processing step can be automatically switched, so that the apparatus can enter the corresponding state in a timely and accurate manner for corresponding processing, ensuring the efficiency of the whole process.

If the target operating state is the preheating state:
At step S223, for example, the value describing the current operating state of the steam ablation apparatus can be set as the status information of the preheating state. In another example, step S223 of determining process can be executed in the filling state, for example. In the filling state, for example, the process of detecting that the current water level is higher than the minimum normal water level (or the maximum normal water level) can be considered as step S223 having been executed.

It can be seen that before step S223, "according to at least one of the current water level, the current steam temperature, and the current pressure, and the current operating state of the steam ablation apparatus, determining the steam ablation apparatus has met the requirements for the target operating state" may specifically include:
When the steam ablation apparatus is in the filling state, detecting that the current water level of the steam generator meets the preheating requirement, which may specifically include detecting that the current water level of the steam generator is higher than or equal to the minimum normal water level. Further, step S222 may be executed when it is detected that the current water level of the steam generator is higher than the minimum normal water level or the maximum normal water level.

In the above technical solution, it is determined whether the water level required for preheating is met, thus providing a basis for the switch of the operating state, and a relatively sufficient amount of water can be ensured when the subsequent heating is started.

If the target operating state is the standby state:
At step S223, for example, the value describing the current operating state of the steam ablation apparatus can be set as the status information of the standby state. In another example, step S223 of determining process can be executed in the preheating state (determining, for example, whether the current water level and current steam temperature of the steam generator meet the requirements for standby).

It can be seen that before step S223, "according to at least one of the current water level, the current steam temperature, and the current pressure, and the current operating state of the steam ablation apparatus, determining the steam ablation apparatus has met the requirements for the target operating state" may specifically include:
When the steam ablation apparatus is in the preheating state, detecting that the current water level and the current steam temperature of the steam generator meet the requirements for standby, which may specifically include detecting that the current water level is higher than or equal to the maximum normal water level and that the current steam temperature is higher than a set disinfection temperature threshold. Furthermore, step S223 may be executed when it is detected that the current water level of the steam generator is higher than the maximum normal water level, and the current steam temperature is higher than the disinfection temperature threshold.

In the above optional solution, it is determined whether the water level and steam requirements for standby are met, thus providing a basis for the switch of the operating state. Also, a sufficient amount of water (that is, higher than the maximum normal water level) and disinfection temperature during subsequent standby are ensured. Based on the water amount, the steam with a required pressure and the supply of the steam in the subsequent process can be ensured. Based on the disinfection temperature, disinfection during standby can be achieved.

If the target operating state is the ablation preparation state:
At step S223, for example, the value describing the current operating state of the steam ablation apparatus can be set as the status information of the ablation preparation state. In another example, step S223 of determining process can be executed in the standby state (determining, for example, whether the current water level of the steam generator meets the requirements for ablation preparation, and whether the steam ablation apparatus has been disinfected treatment).

It can be seen that before step S223, "according to at least one of the current water level, the current steam temperature, and the current pressure, and the current operating state of the steam ablation apparatus, determining the steam ablation apparatus has met the requirements for the target operating state" may specifically include:
When the steam ablation apparatus is in the standby state, detecting that the current water level of the steam generator meets the requirements for ablation preparation, and the steam ablation apparatus has been disinfected treatment, which may specifically include detecting that the current water level is higher than or equal to the maximum normal water level and the steam ablation apparatus has been disinfected treatment. Furthermore, step S223 may be executed when it is detected that the current water level of the steam generator is higher than the maximum normal water level and the disinfection has been completed.

In the above optional solution, it is determined whether the water level required for steam ablation and disinfection are met, thus providing a basis for the switch of the operating state. Also, a sufficient amount of water (that is, higher than the maximum normal water level) and safety during subsequent ablation are ensured.

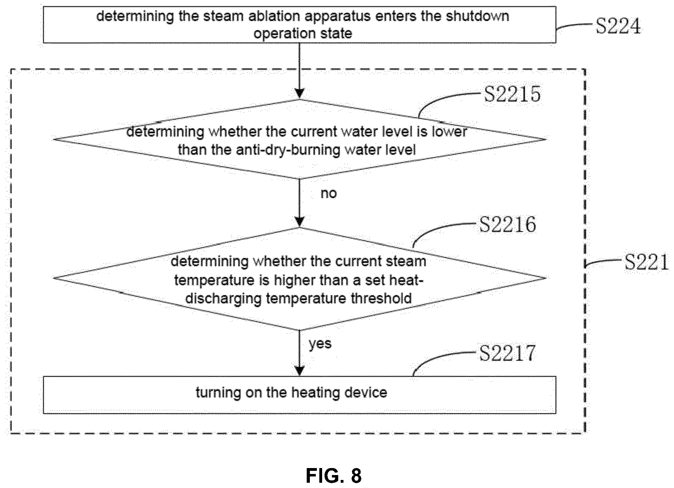

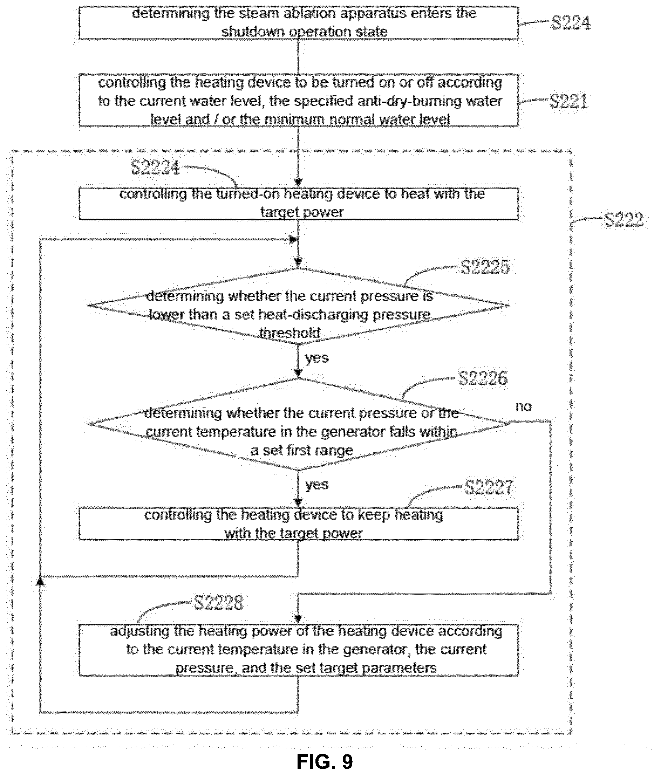

In one embodiment, referring to FIG. 8 and FIG. 9, a specific heating process in the shutdown operation state is shown.

Referring to FIG. 8, step S221 may include:
S2215: determining whether the current water level is lower than the anti-dry-burning water level;
S2216: determining whether the current steam temperature is higher than a set heat-discharging temperature threshold;
When the current water level is higher than or equal to the anti-dry-burning water level, and the current temperature in the generator is higher than the set heat-discharging temperature threshold, step S2217 of turning on the heating device may be executed.

In the above optional solution, heating can be performed in the shutdown state, so as to meet the requirement of heat-discharging (discharging the water in the steam generator by heating) in the shutdown state, so that the water in the steam generator can be discharged quickly in the shutdown state. Also, through the determining of the anti-dry-burning water level, the hidden dangers caused by the low water level can be avoided.

Specifically, referring to FIG. 8, step S222 may include:
S2224: controlling the turned-on heating device to heat with the target power,
S2225: determining whether the current pressure is lower than a set heat-discharging pressure threshold;
S2226: determining whether the current pressure or the current temperature in the generator falls within a set first range;
When the current pressure is lower than the set heat-discharging pressure threshold, and the current pressure or the current temperature in the generator falls within the set first range, step S2227 of controlling the heating device to keep heating with the target power may be executed;
When the current pressure is lower than the set heat-discharging pressure threshold, and the current pressure or the current temperature in the generator falls within a set second range, step S2228 of adjusting the heating power of the heating device according to the current temperature in the generator, the current pressure, and the set target parameters may be executed. The values in the second range are greater than the first range, and the target temperature in the generator or the target pressure falls within the second range.

The above control process based on the first range, the second range, and the target parameters can refer to the relevant description of step S2223. The first range, the second range, and the target parameters used in step S2228 can be the same as or different from those used in step S2223.

In the above optional solution, when the current pressure or the current temperature in the generator is at a small value (such as the first range), all heaters can be controlled to be turned on to reach a higher heating power (such as the maximum heating power), facilitating reaching a greater value (such as the second range) close to the target parameter as soon as possible, and when reaching this range, fine control can be achieved based on the target temperature and target pressure. It can be seen that the above process meets both the efficiency and accuracy requirements for heating.

In one embodiment, since the process shown in FIG. 8 and FIG. 9 are implemented in the shutdown operation state, before step S221, the process may further include:
S224: determining the steam ablation apparatus enters the shutdown operation state.

In the above optional solution, the heating can be started in time after entering the shutdown operation state and meeting the heat-discharging requirement (that is, meeting the steps S2215 and S2216). The heat-discharging process is switched automatically, and the processing efficiency is improved.

At step S224, for example, the value describing the current operating state of the steam ablation apparatus can be set as the status information of the shutdown operation state. In another example, step S224 of determining process can be executed in other states. For example, if an unrecoverable error is detected during the power-on self-test, the steam ablation apparatus may be triggered to shut down, and the process of detecting the unrecoverable error can also be considered as step S224 having been executed.

Further, in the operating states, the above-mentioned processing steps can be implemented by further defining the working states in the operating states.

The above processes controlled based on the specified water levels such as minimum normal water level, maximum normal water level, anti-dry-burning water level, etc., are not limited to the processing method through direct comparison between the specified water level and the current water level. Processing methods by calculation based on, for example, the difference between the current water level and the specified water level, or the ratio of the current water level to the specified water level, or calculation based on the average value of the current water level within a certain duration are not excluded. The same applies to the control based on temperature and pressure. All of these do not depart from the scope of the embodiments of the present disclosure.

Referring to FIG. 10, an embodiment of the present disclosure provides a heating controller 300 for steam ablation apparatus includes:
A monitoring module 301, configured to monitor the current water level in the steam generator, current pressure and current temperature;
A heating control module 302, configured to control the heating device according to the current water level, the current pressure and the current temperature in the generator.

Optionally, the heating control module 302 is specifically configured for:
Controlling the heating device to be turned on or off according to the current water level, and the specified anti-dry-burning water level and / or the minimum normal water level;
After the heating device is turned on, control the heating power of the heating device according to the current pressure and the current temperature in the generator.

Optionally, the heating control module 302 is specifically configured for:
Turning on the heating device if the current water level is higher than or equal to the specified minimum normal water level;
Turning off the heating device if the current water level is lower than the minimum normal water level and higher than or equal to the anti-dry-burning water level.

Optionally, the heating control module 302 is specifically configured for:
Controlling the turned-on heating device to heat with the target power matching the heating power when all heaters of the heating device are turned on when the current pressure or the current temperature in the generator falls within a set first range;
Adjusting the heating power of the heating device according to the current temperature in the generator, the current pressure, and the set target parameters when the current pressure or the current temperature in the generator falls within a second range; the values in the second range are greater than the first range, the target parameters include a target temperature and a target pressure, and the target temperature or the target pressure falls within the second range.

Optionally, referring to FIG. 11, the heating controller 300 further includes:
A state determination module 303, configured to determine that the steam ablation apparatus enters a target operating state; the target operating state is any one of the followings: preheating state, standby state, and ablation preparation state.

Optionally, the monitoring module 301 is further used to monitor the current steam temperature of the steam generator.

Optionally, referring to FIG. 11, the heating controller 300 further includes:
A requirement-met module 304, configured to determine that the steam ablation apparatus has met the requirements for the target operating state according to at least one of the current water level, the current steam temperature, and the current pressure, and the current operating state of the steam ablation apparatus.

If the target operating state is the preheating state:
The requirement-met module 304 is specifically configured for:
Detecting that the current water level of the steam generator meets the requirements for preheating when the steam ablation apparatus is in the filling state.

Optionally, the requirement-met module 304 is specifically configured for:

Detecting that the current water level is higher than the specified minimum normal water level.

Optionally, if the target operating state is the standby state:
The requirement-met module 304 is specifically configured for:
Detecting that the current water level and the current steam temperature of the steam generator meet the requirements for standby when the steam ablation apparatus is in the preheating state.

The requirement-met module 304 is specifically configured for:
Detecting that the current water level is higher than or equal to the specified maximum normal water level, and the current steam temperature is higher than the set disinfection temperature threshold.

Optionally, if the target operating state is the ablation preparation state:
The requirement-met module 304 is specifically configured for:
Detecting that the current water level of the steam generator meets the requirements for steam ablation and the steam ablation apparatus has been disinfected when the steam ablation apparatus is in the standby state.

Optionally, the requirement-met module 304 is specifically configured for:
Detecting that the current water level is higher than or equal to the specified maximum normal water level.

Optionally, the heating control module 302 is specifically configured for:
Turning on the heating device when the current water level is higher than or equal to the anti-dry-burning water level and the current temperature in the generator is higher than the set heat-discharging temperature threshold.

Optionally, the heating control module 302 is specifically configured for:
Controlling the turned-on heating device to heat with the target power matching the heating power when all heaters of the heating device are turned on;
Controlling the heating device to keep heating with the target power when the current pressure is lower than a set heat-discharging pressure threshold and the current pressure or the current temperature in the generator falls within a set first range;
Adjusting the heating power of the heating device according to the current temperature in the generator, the current pressure, and the set target parameters when the current pressure is lower than the set heat-discharging pressure threshold and the current pressure or the current temperature in the generator falls within a set second range; the values in the second range are greater than the first range, and the target temperature in the generator or the target pressure falls within the second range.

Optionally, the heating controller 300 further includes:

A state determination module 303, configured to determine that the steam ablation apparatus enters the shutdown operation state.

Referring to FIG. 12, an electronic device 40 is provided, including:
A processor 41; and
A memory 42, configured to store executable instructions for the processor;
The processor 41 is configured to execute the above-mentioned methods by executing the executable instructions.

The processor 41 is capable of communicating with the memory 42 via a bus 43.

An embodiment of the present disclosure further provides a computer-readable storage medium, in which a computer program is stored, and the above-mentioned methods are implemented when the program is executed by the processor.

Those skilled in the art can understand that all or part of the steps of the above method embodiments can be implemented by program-instruction related hardware. The aforementioned program can be stored in a computer-readable storage medium. When the program is executed, the steps of the above-mentioned method embodiments are implemented; and the aforementioned storage medium includes ROM, RAM, magnetic disk or optical disk and other various media that can store program codes.

Finally, it should be noted that the above embodiments are only intended to explain the technical solutions of the present disclosure, rather than for limitation. Although the present disclosure has been described in detail with reference to the foregoing embodiments, those skilled in the art should understand that it is still possible to modify the technical solutions described in the foregoing embodiments, or make equivalent replacements for some or all of the technical features, and these modifications or replacements do not make the essence of the corresponding technical solutions deviate from the scope of the technical solutions of the various embodiments of the present disclosure.

## Claims

1. A heating control method for steam ablation apparatus, applied to a control device of the steam ablation apparatus, wherein the steam ablation apparatus comprises a steam generator and a heating device, and the heating device is capable of being controlled by the control device and heat the steam generator when the heating device is controlled to be turned on;
the heating control method comprises steps of:
monitoring a current water level in the steam generator, a current pressure and a current temperature in the generator; and
controlling the heating device according to the current water level, the current pressure and the current temperature in the generator.

2. The heating control method of claim 1, wherein,
the step of controlling the heating device according to the current water level, the current pressure and the current temperature in the generator comprises:
controlling the heating device to be turned on or off according to the current water level, and a specified anti-dry-burning water level and / or minimum normal water level; and
controlling a heating power of the heating device according to the current pressure and the current temperature in the generator after the heating device is turned on.

3. The heating control method of claim 2, wherein,
the step of controlling the heating device to be turned on or off according to the current water level, and the specified anti-dry-burning water level and / or minimum normal water level comprises:
turning on the heating device if the current water level is higher than or equal to the specified minimum normal water level; and
turning off the heating device if the current water level is lower than the minimum normal water level and higher than or equal to the anti-dry-burning water level.

4. The heating control method of claim 2, wherein the heating device comprises a plurality of heaters;
the step of controlling the heating power of the heating device according to the current pressure and the current temperature in the generator comprises:
controlling the turned-on heating device to heat with a target power when the current pressure or the current temperature in the generator falls within a set first range; the target power matching a heating power when all heaters of the heating device are turned on; and
adjusting the heating power of the heating device according to the current temperature in the generator, the current pressure and set target parameters when the current pressure or the current temperature in the generator falls within a second range,
wherein values in the second range are greater than the first range, the target parameters comprise a target temperature and a target pressure, and the target temperature or the target pressure falls within the second range.

5. The heating control method of claim 3 or 4, wherein,
before the step of controlling the heating device to be turned on or off according to the current water level, and the specified anti-dry-burning water level and / or minimum normal water level, the method further comprises:
determining that the steam ablation apparatus enters a target operating state being any one of a preheating state, a standby state, and an ablation preparation state.

6. The heating control method of claim 5, further comprising monitoring a current steam temperature of the steam generator;
before the step of determining that the steam ablation apparatus enters the target operating state, the method further comprises:
determining that the steam ablation apparatus has met requirements for the target operating state according to at least one of the current water level, the current steam temperature and the current pressure, and a current operating state of the steam ablation apparatus.

7. The heating control method of claim 6, wherein if the target operating state is the preheating state:
the step of determining that the steam ablation apparatus has met requirements for the target operating state according to at least one of the current water level, the current steam temperature and the current pressure, and the current operating state of the steam ablation apparatus comprises:
detecting that the current water level of the steam generator meets requirements for preheating when the steam ablation apparatus is in a filling state.

8. The heating control method of claim 7, wherein,
the step of detecting that the current water level of the steam generator meets requirements for preheating comprises:
detecting that the current water level is higher than the specified minimum normal water level.

9. The heating control method of claim 6, wherein if the target operating state is the standby state:
the step of determining that the steam ablation apparatus has met requirements for the target operating state according to at least one of the current water level, the current steam temperature and the current pressure, and the current operating state of the steam ablation apparatus comprises:
detecting that the current water level and the current steam temperature of the steam generator meet requirements for standby when the steam ablation apparatus is in the preheating state.

10. The heating control method of claim 9, wherein,
the step of detecting that the current water level and the current steam temperature of the steam generator meet requirements for standby comprises:
detecting that the current water level is higher than or equal to a specified maximum normal water level, and the current steam temperature is higher than a set disinfection temperature threshold.

11. The heating control method of claim 6, wherein if the target operating state is the ablation preparation state:
the step of determining that the steam ablation apparatus has met requirements for the target operating state according to at least one of the current water level, the current steam temperature and the current pressure, and the current operating state of the steam ablation apparatus comprises:
detecting that the current water level of the steam generator meets requirements for steam ablation and the steam ablation apparatus has been disinfected when the steam ablation apparatus is in the standby state.

12. The heating control method of claim 11, wherein:
the step of detecting that the current water level of the steam generator meets requirements for steam ablation comprises:
detecting that the current water level is higher than or equal to a specified maximum normal water level.

13. The heating control method of claim 2, wherein:
the step of controlling the heating device to be turned on or off according to the current water level, and the specified anti-dry-burning water level and / or minimum normal water level comprises:
turning on the heating device when the current water level is higher than or equal to the anti-dry-burning water level and the current temperature in the generator is higher than a set heat-discharging temperature threshold.

14. The heating control method of claim 2, wherein the heating device comprises a plurality of heaters;
the step of controlling the heating power of the heating device according to the current pressure and the current temperature in the generator comprises:
controlling the turned-on heating device to heat with a target power matching a heating power when all heaters of the heating device are turned on;
controlling the heating device to keep heating with the target power when the current pressure is lower than a set heat-discharging pressure threshold and the current pressure or the current temperature in the generator falls within a set first range; and
adjusting the heating power of the heating device according to the current temperature in the generator, the current pressure and set target parameters when the current pressure is lower than the set heat-discharging pressure threshold and the current pressure or the current temperature in the generator falls within a set second range;
values in the second range are greater than the first range, and a target temperature in the generator or a target pressure falls within the second range.

15. The heating control method of claim 13 or 14, wherein,
before the step of controlling the heating device to be turned on according to the current water level and the specified anti-dry-burning water level, the method further comprises:
determining that the steam ablation apparatus enters a shutdown operation state.

16. The heating control method of any one of claims 1 to 4, 13 and 14, wherein the steam ablation apparatus has a plurality of operating states, and the plurality of operating states comprise filling state, preheating state, standby state, ablation preparation state and shutdown operation state; the steam ablation apparatus is capable of entering the filling state, the preheating state, the standby state and the ablation preparation state sequentially.

17. A heating controller for steam ablation apparatus, applied to a control device of the steam ablation apparatus, wherein the steam ablation apparatus comprises a steam generator and a heating device, and the heating device is configured to be controlled by the control device and heat the steam generator when the heating device is controlled to be turned on;
the heating controller comprises:
a monitoring module, configured to monitor a current water level in the steam generator,
a current pressure and a current temperature in the generator; and
a heating control module, configured to control the heating device according to the current water level, the current pressure and the current temperature in the generator.

18. A steam ablation apparatus, comprising a steam generator, a heating device and a control device, the heating device is configured to be controlled by the control device and heat the steam generator when the heating device is controlled to be turned on, and the control device is configured to execute the heating control method of any one of claims 1 to 16.

19. An electronic device, comprising a processor and a memory, wherein
the memory is configured to store codes; and
the processor is configured to execute the codes in the memory to implement the heating control method of any one of claims 1 to 16.

20. A storage medium, in which a computer program is stored, and when the program is executed by a processor, the heating control method of any one of claims 1 to 16 is implemented.
